# EUROPEAN PATENT APPLICATION

(11) **EP 1 480 040 A2**
(43) Date of publication of application: **24.11.2004**
(21) Application number: 04380112.5
(22) Date of filing: 21.05.2004
(51) Int. Cl.: G01N 29/04, G01N 29/08, G01N 29/10, G01N 33/38, G01N 33/24

(54) **Procedure to diagnose the quality in blocks of ornamental rock of large dimensions and devices for its implementation**

(30) Priority: 23.05.2003 ES 200301207
(71) Applicant: Asociacion de Investigacion de las Industrias de la Construccion (AIDICO), 46980 Paterna (Valencia) (ES)
(72) Inventor: The designation of the inventor has not yet been filed
(74) Representative: Munoz Garcia, Antonio

(57) **Abstract**

The object of this Patent is a "Procedure to diagnose the quality, in blocks of ornamental rock of large dimensions, and devices for its implementation", whose main use is explicit in the statement of the invention itself.

The claimed procedure comprises three inspection techniques, successively applicable, which respond to increasing precisions in the diagnosis, and establishing a norm of action in order to know whether the block may be cut into sections, or if it shall be considered for rejection from the process, complementing the stage of primary diagnosis with a more detailed inspection, through the carrying out of ultrasonic tomographies for the location of the fractures and in that case, of their orientation, from the spread of signals in different points of a mesh made on the surface of its sides and their digital processing.

## Description

### OBJECT OF THE INVENTION

The invention protected in this Patent refers to an object, which consists of a "Procedure to diagnose the quality, in blocks of ornamental rock of large dimensions and devices for its implementation".

### BACKGROUND.

One of the main problems for the quarries and the ornamental rock processing factories, lies in the fact that the perception of whether a block is good or not is subjective, subject to the experience of the worker and there is usually an error in its verdict. In fact, a block may have a good outer appearance, assuming the inexistence of discontinuities in its interior, and yet, later, when cutting it into sections in order to obtain slabs and tiles, discontinuities may be found, with inevitable loss of material, risk for the worker and the possibility of breakage of the cutting wire.

Although the application of non-destructive methods through ultrasounds is common practice for natural stone, there is no systematic procedure to diagnose blocks of ornamental rock.

The rudimentary method used by the quarry workers to know whether a rock block is internally fractured, entails the worker tapping specifically with a quarry worker mace on the surface of the sides keeping the other hand around 20-30 centimetres from the tapping area. Taking into account the sound of the impact and the vibration he feels on his hand, he decides the state of that area and the presence or lack of defects in its interior. The low sounds (muffled) which are right away reduced show a poor quality or the presence of internal fracture. The appearance of very high frequencies may be indicative of the presence of laminates near the surface. The method does not entail any associated technology, it is subjective and its reliability depends on the training of the worker.

Within the application in ornamental rock, one of the most well-known techniques that suits the block diagnosis best, consists in the analysis of sonic waves collected in piece-electric sensors. This technique facilitates the analysis of the quality of the block through a few measurements of the speed of sound in the same direction, but at points on two opposite sides of the block. The decrease in speed of one point, shows the presence of an error between the point that emits and the one that receives, in such a way that the larger the mesh, that is to say, the number of measurement points, the more precise the method will be; but it does not establish a norm to make that mesh meet the dimensions of the block, in order to get an optimum resolution, and so it does not allow to obtain an assessment that identifies and locates the defects in the blocks of rock.

### DESCRIPTION OF THE INVENTION.

The purpose of the invention that is the object of this Patent, consists of comprising three inspection techniques, successively applicable, which respond to increasing precisions in the diagnosis, and establishing a norm of action in order to know whether the block may be cut into sections, or if it shall be considered for rejection from the process, complementing the stage of primary diagnosis with a more detailed inspection, through the carrying out of ultrasonic tomographies for the location of the fractures and in that case, of their orientation, from the spread of signals in different points of a mesh made on the surface of its sides and their digital processing.

There are characteristics to be taken into account because they affect the precision of the procedure, such as the shape of the block or imbalance and the appearance of their sides, which should be smooth, parallel and clean.

An effective fitting between the sensors and the block, facilitates the of obtaining better tomographies, which requires this fitting to be as uniform as possible in all the measurements

The three diagnosis techniques comprised in the claimed procedure, are the following :

### 1) - By hammer impact (MIM).

It comprises of the following operations, carried out with the same sequence as described:
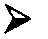 Placing the block on prismatic and homogeneous pieces of wood.
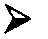 Placing a sensor in the geometric centre of the side that is tapped with the hammer and a receiver in the geometric centre of each of the other accessible sides. In case the sides are not regular, they shall be placed in the nearest location that allows an optimum fitting.
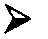 Tapping with a hammer in a place near the sensor of that side, which shall be used as an outward shot of a data collection card and as a signal to enter the system, in order to inform about the frequency content introduced as a mechanic thrust.
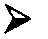 Introducing the signals collected in each channel into a computer, so that the algorithms to obtain the parameters that characterise the quality of the block can be carried out.
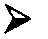 Introducing into the computer intrinsic parameters of the block (dimensions, type of ornamental rock) and the position of the sensors in respect of the place of impact, which are used by the logarithm in charge of the classification.
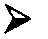 Reiterating the process of collection of signals and calculating the variance of the signals in a certain period of time and frequency, carrying out an algorithm prior to that of the classification, which informs about the validity of the measurements taken from the block, through the variance of the data in each channel. This first test of the signals analyses their possibility to be included either in a database to prepare a classification algorithm, or in a data base for future reference.
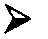 Determining from the pieces of information included in the database, which parameters shall carry more weigh for the calculation, depending on the type of rock.
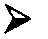 Activating the classification algorithm, using the information obtained in the previous operations.
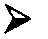 Diagnosing the state of the block, taking into account even the slightest deviation of the parameters in respect of the vectors of parameters corresponding to the type of rock, this operation is carried out by the classification algorithm.
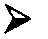 Identifying deviations attributable to the existence of internal defects, in the direction that links the place of impact and the place of the channel reception, to determine the most appropriate orientation for the cut, in order to minimise the loss of material.
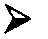 Introduce the vectors of correctly classified parameters into the database again.

The necessary equipment for the implementation of this technique comprises:
- A Hammer for the analysis of structures, with a steel head.
- Sensors of signal or vibration (low frequency signal transducers or mono-dimensional accelerometers).
- An Oscilloscope of at least 2 channels, in order to capture the vibration near the hammer tapping and the one registered on the other sides of the block.
- Data collection card.
- A System of external shot with operation control via software.
- An Amplifier of the digital signals in each channel.
- A PC to control via software the collection of data in order to process them digitally.

The MIM technique facilitates the classification of the blocks according to their general state in :
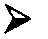 Sound blocks : They do not show significant defects which may cause considerable losses of material when sawing the block up. These blocks may go directly to the sawing into sections, without it being necessary to pre-determine the position in which they shall be cut.
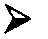 Regular blocks : There are doubts about the non-existence of defects, so it is convenient to continue the procedure of diagnosis.
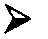 Blocks in bad condition : They show defects that may cause losses of material or general multi-fracture.

Those blocks classed as regular or in bad condition by the technique of diagnosis through impact, the claimed procedure proposes that they shall be diagnosed through a technique with higher resolution, in order to locate the internal defects, so they shall go, in the first place, to the diagnosis through the following associated technique.

### 2) - Low Resolution (MBR).

After the diagnosis through the MIM technique, and in case there is the presumption that the block may have any defect in its interior, or a imperceptible fissure level, the claimed procedure plans to submit it to a more effective inspection technique.

This second technique of the claimed procedure consists of:
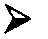 Making a mesh of points on opposite sides of the block, in longitudinal and transversal directions, lining each one of them up with its corresponding one on the opposite side, depending on the separation between the points of frequency used and of the diagram of radiation of the ultrasound transducers used.
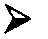 Carrying out measurements of transmission-reception in opposite sides with two transducers of the same characteristics (frequency, width of band and diagram of radiation) lined up, obtaining a signal from every point of the mesh of one of the sides.
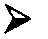 Transferring the ultrasonic signals from the mesh points, in an orderly way, onto a 3D matrix, in order to know to which point (position) each collected signal corresponds to.
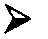 Digital processing of the signals corresponding to a direction, in order to get a parameter (longitudinal speed, or reduction of the P wave of arrival) for each one of the signals, from which 2D maps or tomographies are carried out that represent the quality of the block and its state of internal fissure.

The necessary equipment for the implementation of this technique comprises of the following:
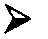 Ultrasound equipment, a transmitter-receiver with a pulse of adjustable high voltage and with a reception stage with a high gain.
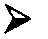 Two transducers of similar characteristics, of low nominal frequency and with a directing radiation diagram.
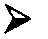 Co-axial cables of radio-frequency of 75-ohm impedance.
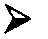 A means for fitting by contact, using a high density non greasy gel, or by injected water in a laminar pattern, in order not to affect the ultrasonic beam which shall spread through it or by immersion of the signal transducer.

In the event that the anomalous areas are numerous or that the tomographies show speed values below 60% of the usual one in a wide area, the block is considered as a rejection.

When the 2D maps detect located areas with a significant decrease in the speed parameters or increase in the values of the reduction reflected in the tomographies, the procedure advices you to make a high resolution mesh for the detection of the defect, placing it on the inside of the block.

### 3) - High Resolution (MAR).

This third technique of the claimed procedure consists of the following :
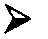 Making a mesh of points on one side of the block, whose separation shall depend on the frequency used and on the radiation diagram of the ultrasound transducers used.
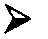 Obtaining measurements through pulse/echo in each one of the points of the mesh, attacking the side of the block which is assumed to be closer to the anomalous area detected in the 2D tomography.
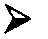 Transferring the ultrasonic signals of pulse/echo to each one of the mesh points, in an orderly way, onto a 3D matrix by rows and columns, which shall correspond with the mesh positions, specified also by rows and columns.
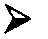 Digital processing of the signals to show the positions where reflections occur and their representation in a internal 3D map of defects, successively carrying out, for each signal, the pre-processing algorithms, detection by threshold and representation.

The necessary equipment for the implementation of this technique comprises :
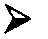 Ultrasound equipment, transmitter-receiver with a pulse of adjustable high voltage and with a reception stage with a high gain.
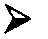 Two transducers of similar characteristics, of low nominal frequency and with a directing radiation diagram.
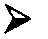 Co-axial cables of radio-frequency of 75-ohm impedance.
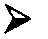 A Means for fitting by contact, using a high density non greasy gel, or by injected water in laminar a pattern, in order not to affect the ultrasonic beam which shall spread through it or by immersion of the signal transducer.

### BRIEF DESCRIPTION OF THE FIGURES.

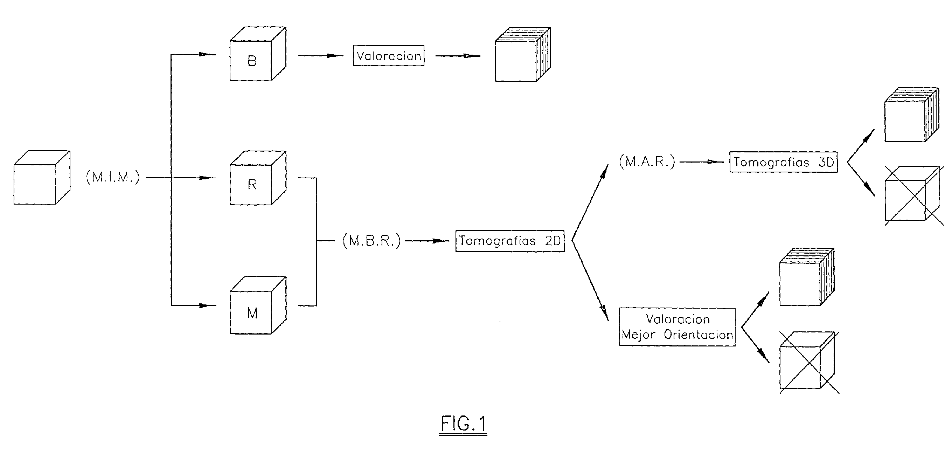

In order to complement the description of the invention and facilitate the understanding and the follow-up of the operations comprised in the "Procedure to diagnose the quality, in blocks of ornamental rock of large dimensions" that constitute its object, Figure 1 is enclosed as a sequential outline of the aforementioned operations, which shall be taken as an indicative guide of its preferential development.

### DESCRIPTION OF A PREFERENTIAL EMBODIMENT.

In order to show clearly the nature and scope of the advantageous application of the "Procedure to diagnose the quality, in blocks of ornamental rock of large dimensions" which constitutes the object of the claimed invention, the operations that it consists of and their sequence are next described referring to the outline that, since it represents a preferential embodiment of the aforementioned procedure, of an informative character, it should be considered in its widest meaning and not as a limit to the application and the content of the claimed invention.

The procedure comprises of three inspection techniques, successively applicable, which respond to increasing precisions in the diagnosis, and establishing a norm of action in order to know whether the block may be cut into sections, or if it shall be considered for rejection from the process, complementing the stage of primary diagnosis with a more detailed inspection, through the carrying out of ultrasonic tomographies for the location of the fractures and in that case, of their orientation, from the spread of signals in different points of a mesh made on the surface of its sides and their digital processing.

The first one of the techniques applied for its development, called "Method of hammer impact" (MIM), comprises of the following operations, carried out in the same sequence as described:
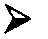 Placing the block on prismatic and homogeneous pieces of wood.
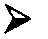 Placing a sensor in the geometric centre of the side that is tapped with the hammer and each receiver in the geometric centre of the other accessible sides. In case the sides are not regular, they shall be placed in the nearest location that allows an optimum fitting.
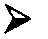 Tapping with a hammer in a place near the sensor of that side, which shall be used as an outward shot of a data collection card and as a signal to enter the system, in order to inform about the frequency content introduced as a mechanic thrust.
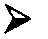 Introducing the signals collected in each channel into a computer, so that the algorithms to obtain the parameters that characterise the quality of the block can be carried out.
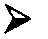 Introducing into the computer the intrinsic parameters of the block (dimensions, type of ornamental rock) and the position of the sensors in respect of the place of impact, which are used by the logarithm in charge of the classification.
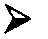 Reiterating the process of collection of signals and calculating the variance of the signals in a certain period of time and frequency, carrying out an algorithm prior to that of the classification, which informs about the validity of the measurements taken from the block, through the variance of the data in each channel. This first test of the signals analyses their possibility to be included either in a database in order to prepare a classification algorithm, or else in a data base for future reference.
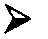 Determining from the pieces of information included in the database, which parameters shall carry more weigh for the calculation, depending on the type of rock.
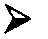 Activating the classification algorithm, using the information obtained in the previous operations.
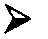 Diagnosing the state of the block, taking into account even the slightest deviation of the parameters in respect of the vectors of the parameters corresponding to the type of rock, this operation is carried out by the classification algorithm.
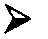 Identifying deviations attributable to the existence of internal defects, in the direction that links the place of impact and the place of the channel reception, to determine the most appropriate orientation for the cut, in order to minimise the loss of material.
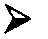 Introduce the vectors of correctly classified parameters into the database again.

The device for the implementation of the first of the techniques related to the claimed procedure (MIM), comprises of the following equipment:
- A Hammer for the analysis of structures, with a steel head.
- Sensors of signal or vibration (low frequency signal transducers or mono-dimensional accelerometers).
- An Oscilloscope of at least 2 channels, in order to capture the vibration near the hammer tapping and the one registered on the other sides of the block.
- Data collection card.
- A System of external shot with operation control via software.
- An Amplifier of the digital signals in each channel.
- A PC to control via software the collection of data in order to process them digitally.

The application of the MIM technique facilitates the classification of the blocks according to their general state in :
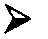 Sound blocks : They do not show significant defects which may cause considerable loss of material when sawing the block up. These blocks may go directly to the sawing into sections, without it being necessary to pre-determine the position in which they shall be cut.
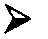 Regular blocks : There are doubts about the non-existence of defects, so it is convenient to continue the procedure of diagnosis.
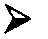 Blocks in bad condition : They show defects that may cause loss of material or general multi-fracture.

Those blocks classed as regular or in bad condition by the technique of diagnosis through impact; the claimed procedure anticipates that they shall be diagnosed through a technique with higher resolution, in order to locate the internal defects, so they shall go, in the first place, to the diagnosis through the second associated technique.

The second of the applied techniques for its development, called "Method of low resolution" (MBR), comprises of the following operations carried out in the same sequence as described:
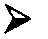 Making a mesh of points on opposite sides of the block, in longitudinal and transversal directions, lining each one of them up with its corresponding one on the opposite side, depending on the separation between the points of frequency used and of the diagram of radiation of the ultrasound transducers used.
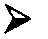 Carrying out measurements of transmission-reception on opposite sides with two transducers of the same characteristics (frequency, width of band and diagram of radiation) lined up, obtaining a signal from every point of the mesh of one of the sides.
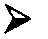 Transferring the ultrasonic signals from the mesh points, in an orderly way, onto a 3D matrix, in order to know to which point (position) each collected signal corresponds to.
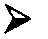 Digital processing of the signals corresponding to a direction, in order to get a parameter (longitudinal speed, or reduction of the P wave of arrival) for each one of the signals, from which 2D maps or tomographies are carried out that represent the quality of the block and its state of internal fissure.

The device for the implementation of the second technique related to the claimed procedure (MBR) comprises of the following equipment:
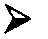 Ultrasound equipment, transmitter-receiver with a pulse of adjustable high voltage and with a reception stage with a high gain.
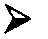 Two transducers of similar characteristics, of low nominal frequency and with a directing radiation diagram.
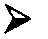 Co-axial cables of radio-frequency of 75-ohm impedance.
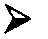 A Means for fitting by contact, using a high density non greasy gel, or by injected water in a laminar pattern, in order not to affect the ultrasonic beam which shall spread through it or by immersion of the signal transducer.

The third of the techniques applied for its development, called "Method of high resolution" (MAR), comprises of the following operations carried out in the same sequence as described:
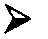 Making a mesh of points on one side of the block, whose separation shall depend on the frequency used and on the radiation diagram of the ultrasound transducers used.
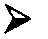 Obtaining measurements through pulse/echo in each one of the points of the mesh, attacking the side of the block which is assumed to be closer to the anomalous area detected in the 2D tomography.
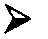 Transferring the ultrasonic signals of pulse/echo to each one of the mesh points, in an orderly way, onto a 3D matrix by rows and columns, which shall correspond with the mesh positions, specified also by rows and columns.
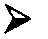 Digital processing of the signals to show the positions where reflections occur and their representation in a internal 3D map of defects, successively carrying out, for each signal, the pre-processing algorithms, detection by threshold and representation.

The device for the implementation of the third of the Techniques related to the claimed procedure (MAR), comprises of the following equipment:
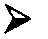 Ultrasound equipment, transmitter-receiver with a pulse of adjustable high voltage and with a reception stage with a high gain.
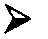 Two transducers of similar characteristics, of low nominal frequency and with a directing radiation diagram.
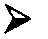 Co-axial cables of radio-frequency of 75-ohm impedance.
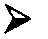 A Means for fitting by contact, using a high density non greasy gel, or by injected water in a laminar pattern, in order not to affect the ultrasonic beam which shall spread through it or by immersion of the signal transducer.

## Claims

1. Procedure to diagnose the quality, in blocks of ornamental rock of large dimensions, of the type that is developed through the analysis of sonic waves collected in piece-electric sensors, **characterised by** comprising three inspection techniques, successively applicable, which respond to increasing precisions in the diagnosis, and establishing a norm of action in order to know whether the block may be cut into sections, or if it shall be considered for rejection from the process, complementing the stage of primary diagnosis with a more detailed inspection, through the carrying out of ultrasonic tomographies for the location of the fractures and in that case, of their orientation, from the spread of signals in different points of a mesh made on the surface of its sides and their digital processing.

2. Procedure to diagnose the quality, in blocks of ornamental rock of large dimensions, according to Claim 1, **characterised because** the first of the techniques applied for its development, called "Method of hammer impact" (MIM), comprises of the following operations, carried out in the same sequence as described:
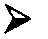 Placing the block on prismatic and homogeneous pieces of wood.
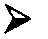 Placing a sensor in the geometric centre of the side that is tapped with the hammer and a receiver in the geometric centre of each of the other accessible sides. In case the sides are not regular, they shall be placed in the nearest location that allows an optimum fitting.
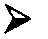 Tapping with a hammer in a place near the sensor of that side, which shall be used as an outward shot of a data collection card and as a signal to enter the system, in order to inform about the frequency content introduced as a mechanic thrust.
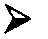 Introducing the signals collected in each channel into a computer, so that the algorithms to obtain the parameters that characterise the quality of the block can be carried out.
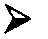 Introducing into the computer intrinsic parameters of the block (dimensions, type of ornamental rock) and the position of the sensors in respect of the place of impact, which are used by the logarithm in charge of the classification.
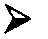 Reiterating the process of collection of signals and calculating the variance of the signals in a certain period of time and frequency, carrying out an algorithm prior to that of the classification, which informs about the validity of the measurements taken from the block, through the variance of the data in each channel. This first test of the signals analyses their possibility to be included either in a database in order to prepare a classification algorithm, or in a data base for future reference.
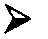 Determining from the pieces of information included in the database, which parameters shall carry more weigh for the calculation, depending on the type of rock.
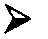 Activating the classification algorithm, using the information obtained in the previous operations.
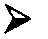 Diagnosing the state of the block, taking into account even the slightest deviation of the parameters in respect of the vectors of the parameters corresponding to the type of rock, this operation is carried out by the classification algorithm.
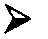 Identifying deviations attributable to the existence of internal defects, in the direction that links the place of impact and the place of the channel reception, to determine the most appropriate orientation for the cut, in order to minimise the loss of material.
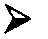 Introduce the vectors of correctly classified parameters into the database again.

3. Device for the implementation of the first of the techniques related to the claimed procedure (MIM), **characterised because** it comprises of the following equipment :
- A Hammer for the analysis of structures, with a steel head.
- Sensors of signal or vibration (low frequency signal transducers or mono-dimensional accelerometers).
- An Oscilloscope of at least 2 channels, in order to capture the vibration near the hammer tapping and the one registered on the other sides of the block.
- Data collection card.
- A System of external shot with operation control via software.
- An Amplifier of the digital signals in each channel.
- A PC to control via software the collection of data in order to process them digitally.

4. Procedure to diagnose the quality, in blocks of ornamental rock of large dimensions, according to Claim 2, **characterised because** the application of the MIM technique facilitates the classification of the blocks according to their general state in :
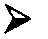 Sound blocks : They do not show significant defects which may cause considerable loss of material when sawing the block up. These blocks may go directly to the sawing into sections, without it being necessary to pre-determine the position in which they shall be cut.
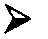 Regular blocks : There are doubts about the non-existence of defects, so it is convenient to continue the procedure of diagnosis.
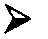 Blocks in bad condition : They show defects that may cause loss of material or general multi-fracture.
Those blocks classed as regular or in bad condition by the technique of diagnosis through impact; the claimed procedure anticipates that they shall be diagnosed through a technique with higher resolution, in order to locate the internal defects, so they shall go, in the first place, to the diagnosis through the second associated technique.

5. Procedure to diagnose the quality, in blocks of ornamental rock of large dimensions, according to Claim 1, **characterised because** the second of the applied techniques for its development, called "Method of low resolution" (MBR) , comprises of the following operations carried out in the same sequence as described:
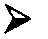 Making a mesh of points on opposite sides of the block, in longitudinal and transversal directions, lining each one of them up with its corresponding one on the opposite side, depending on the separation between the points of frequency used and of the diagram of radiation of the ultrasound transducers used.
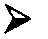 Carrying out measurements of transmission-reception on opposite sides with two transducers of the same characteristics (frequency, width of band and diagram of radiation) lined up, obtaining a signal from every point of the mesh of one of the sides.
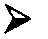 Transferring the ultrasonic signals from the mesh points, in an orderly way, onto a 3D matrix, in order to know to which point (position) each collected signal corresponds to.
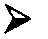 Digital processing of the signals corresponding to a direction, in order to get a parameter (longitudinal speed, or reduction of the P wave of arrival) for each one of the signals, from which 2D maps or tomographies are carried out that represent the quality of the block and its state of internal fissure.

6. Device for the implementation of the second of the techniques related to the claimed procedure (MBR), **characterised because** it comprises of the following equipment:
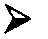 Ultrasound equipment, a transmitter-receiver with a pulse of adjustable high voltage and with a reception stage with a high gain.
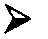 Two transducers of similar characteristics, of low nominal frequency and with a directing radiation diagram.
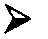 Co-axial cables of radio-frequency of 75-ohm impedance.
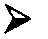 A Means for fitting by contact, using a high density non greasy gel, or by injected water in a laminar pattern, in order not to affect the ultrasonic beam which shall spread through it or by immersion of the signal transducer.

7. Procedure to diagnose the quality, in blocks of ornamental rock of large dimensions, according to Claim 1, **characterised because** the third of the techniques applied for its development, called "Method of high resolution" (MAR), comprises of the following operations carried out in the same sequence as described:
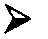 Making a mesh of points on one side of the block, whose separation shall depend on the frequency and on the radiation diagram of the used ultrasound transducers used.
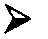 Obtaining measurements through pulse/echo in each one of the points of the mesh, attacking the side of the block which is assumed to be closer to the anomalous area detected in the 2D tomography.
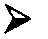 Transferring the ultrasonic signals of pulse/echo to each one of the mesh points, in an orderly way, onto a 3D matrix by rows and columns, which shall correspond with the mesh positions, specified also by rows and columns.
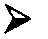 Digital processing of the signals to show the positions where reflections occur and their representation in a internal 3D map of defects, successively carrying out, for each signal, the pre-processing algorithms, detection by threshold and representation.

8. Device for the implementation of the third of the Techniques related to the claimed procedure (MAR), **characterised because** it comprises of the following equipment:
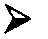 Ultrasound equipment, a transmitter-receiver with a pulse of adjustable high voltage and with a reception stage with a high gain.
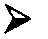 Two transducers of similar characteristics, of low nominal frequency and with a directing radiation diagram.
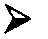 Co-axial cables of radio-frequency of 75-ohm impedance.
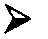 A Means for fitting by contact, using a high density non greasy gel, or by injected water in a laminar pattern, in order not to affect the ultrasonic beam which shall spread through it or by immersion of the signal transducer.
